(19) Europäisches Patentamt / European Patent Office / Office européen des brevets

(11) **EP 4 520 262 A1**

(12) **EUROPEAN PATENT APPLICATION**

(43) Date of publication:
**12.03.2025 Bulletin 2025/11**

(21) Application number: **23196254.9**

(22) Date of filing: **08.09.2023**

(51) International Patent Classification (IPC):
**A61B 5/053** (2021.01)     **A61B 5/113** (2006.01)
**A61B 5/00** (2006.01)

(52) Cooperative Patent Classification (CPC):
**A61B 5/1135; A61B 5/053; A61B 5/6805**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Nanoleq AG**
**8153 Rümlang (CH)**

(72) Inventors:
• **Stauffer, Flurin**
  **Zürich (CH)**
• **Torvinen, Vesa-Pekka**
  **Siivikkala (FI)**
• **Weydert, Serge**
  **Kloten (CH)**
• **Martinez, Vincent**
  **Zürich (CH)**

(74) Representative: **Vossius & Partner**
**Patentanwälte Rechtsanwälte mbB**
**Siebertstrasse 3**
**81675 München (DE)**

(54) **DEVICE FOR SENSING A USER'S MOTION OF THE USER'S CHEST AND ABDOMEN**

(57)    The invention relates to respiration sensing, in particular to sensing chest and abdominal breathing. Furthermore, the invention relates to sensing a user's motion of the user's chest and abdomen. In particular, the invention relates to a device for sensing a user's motion of the user's chest and abdomen.

Fig. 1

**Description**

The invention relates to respiration sensing, in particular to sensing chest and abdominal breathing. Furthermore, the invention relates to sensing a user's motion of the user's chest and abdomen. In particular, the invention relates to a device for sensing a user's motion of the user's chest and abdomen.

It is known that a user's motion of the chest and/or abdomen can be measured, e.g., by respiratory inductance plethysmography (RIP). A respiratory inductance plethysmograph or RIP band may comprise an electrical conductor configured to surround the user's chest thereby forming a coil. During inspiration, the cross-sectional area of the rib cage and abdomen of the user increases, thereby altering the inductance of the coil. The movement of the user's chest and/or abdomen can thus be sensed by measuring the inductance of the coil.

It is also known to provide the user with a first RIP surrounding the chest and a second RIP surrounding the abdomen. Such an arrangement is, e.g., shown in Fig. 2 of GB1596298A. Furthermore, Fig. 1 of GB1596298A shows an extensible electrical conductor multi-turn loop attached to a tubular stretch bandage in the form of a sleeveless sweater worn by the patient, so that the multi-turn loop is held in close encirclement about the patient's torso while the patient breathes.

An object of the present invention is to provide a respiration sensor solution that is capable of efficiently capturing both the thorax and abdominal respiration. A further object of the invention is to capture the thorax and abdominal respiration with only one sensor channel. Further objects of the invention are to improve the sensitivity of the respiration sensor solution, reduce motion artifacts, tune the signal contribution between thorax and abdominal respiration, and provide for a calibration of respiration signals. Additional objects of the invention are to improve the wear comfort of the respiration sensor solution and simplify the manufacturing of the respiration sensor solution.

These and further objects are achieved by the subject-matter of the independent claims.

A first aspect of the invention relates to a device for sensing a user's motion of the user's chest and abdomen, comprising a garment configured to be worn on the upper body by the user, and a sensor configured to sense the expansion and/or contraction of the user's chest and abdomen, the sensor being attached to the garment. The sensor has a figure eight configuration with a first loop of the sensor being configured to at least partly extend over and/or surround the user's chest, a second loop of the sensor configured to at least partly extend over and/or surround the user's abdomen. Preferably, the device and/or garment is configured to be worn on the upper body of the user such that a crossing point between the first loop and the second loop is located at the user's back.

Such a device enables measuring the breathing rate, breathing patterns, breathing interruptions, tidal volume, inhale time, exhale time, and/or further breathing-related parameters of a user. By providing the figure eight configuration with a first loop and a second loop, the device enables sensing chest breathing and abdominal breathing. Moreover, the sensitivity of the device may be improved and/or motion artifacts in the signal generated by the device may be reduced.

Preferably, the garment is configured to be worn at least partly on the user's chest and abdomen. Preferably, the garment comprises an elastic and/or stretchable material, such that the garment is configured to closely fit and/or conform to the upper body of the user. For example, the garment may be provided in the form of sports underwear. In this way, the first loop and the second loop can be securely positioned on the user's chest and abdomen, respectively.

The term 'sensor' may refer to a structure that is attached to the garment and that forms and/or comprises a first loop and a second loop arranged in a figure eight configuration, the first loop being configured to at least partially extend over and/or surround the user's chest and the second loop being configured to at least partially extend over and/or surround the user's abdomen. The sensor may comprise an elongated electrical conductor. The expansion and/or contraction of the user's chest may influence an electrical property of the electrical conductor, such as an inductance of a coil formed by the electrical conductor, a resistance of the electrical conductor, and/or a capacity of the electrical conductor. The electrical conductor may be an extensible conductor, e.g., as disclosed in more detail further below. Alternatively or additionally, the electrical conductor may be arranged in in sinusoidal or zig-zag pattern on an extensible portion of the garment. In the latter case, the electrical conductor does not necessarily need to be extensible.

For example, the sensor may be an elongated elastic seam tape as described, e.g., in claims 1 to 14 of WO 2021/09428 A1. Such an elongated elastic seam tape may comprise an elongated elastic first layer, an elongated elastic second layer partially bonded to the first layer, and an elongated elastic conductor, wherein each longitudinal edge of the second layer is bonded to a corresponding longitudinal edge of the first layer so as to form a lumen extending along the entire seam tape between the first and second layers. Furthermore, the conductor is movably positioned within said lumen. Once the seam tape is fully bonded to the garment, the first layer and the second layer may fit tight to the conductor. That is, the conductor may fully occupy the space between the first and second layer. However, the first and second layers do not necessarily have to fit tight to the conductor and there may be additional space between the first layer and the second layer that is not fully occupied by the conductor.

The feature that the first loop is configured to at least partially extend over and/or surround the user's chest and the second loop is configured to at least partially extend over and/or surround the user's abdomen may be understood to specify that the first loop and the second loop are arranged on the garment in such a way that, when the user is wearing the garment, the first loop at least partially extends over and/or surrounds the user's chest and the second loop at least partially extends over and/or surrounds the user's abdomen. Preferably, the first loop is configured to at least partially or fully surround the user's chest and the second loop is configured to at least partially or fully surround the user's abdomen.

The first loop and the second loop of the sensor are arranged in a figure eight configuration. As such, the sensor may be arranged on the garment in such a way that the sensor forms a first loop and a second loop, wherein the first loop adjoins the second loop at a crossing point.

The crossing point may refer to the location where a first portion of the sensor crosses and/or overlaps a second portion of the sensor. In other words, the sensor itself may be arranged in such a way that it forms a figure eight shape with a first loop and a second loop when laid out flat. However, in the arrangement of the sensor on the garment, the first loop or the second loop may be folded over towards the other loop, such that the user's upper body can pass through the first loop and the second loop. Thus, when the sensor is arranged on the garment, it may not be possible to lay the sensor out flat.

Preferably, the sensor does not comprise a further loop in addition to the first loop and the second loop. In other words, the sensor may be understood to consist of the first loop and the second loop.

Providing the sensor in a figure eight configuration with a first loop configured to at least partly extend over the chest and a second loop configured to at least partly extend over the abdomen, the sensor may be sensitive to chest breathing and to abdominal breathing. Furthermore, by arranging the sensor such that it has two loops, the working voltage may be improved and the overall respiration signal amplitude may be increased, which may make the device more robust. Furthermore, changes in the first and second loop that may be caused by body movements that are not caused by breathing and/or by the movement of air from the chest to the abdomen or vice versa without breathing may cancel each other at least partially. Therefore, such an arrangement with a first loop and a second loop may reduce signal artifacts that may be caused by body movements. Moreover, providing the sensor in form of a figure eight configuration may simplify arrange the sensor on the garment, while ensuring that the sensor comprises a first loop configured to at least partly extend over the chest and a second loop configured to at least partly extend over the abdomen.

Preferably, the crossing point between the first loop and the second loop is configured to be arranged on the user's back, when the garment is worn by the user.

Thus, the first loop may extend at least over a ventral region of the user's chest and the second loop may extend at least over a ventral region of the user's abdomen. In this way, the sensitivity of the sensor to breathing movement may be improved. Furthermore, by providing the crossing point on the user's back, the wearing comfort of the device may be improved.

The device of the first aspect may optionally further comprise a first capacitive plate or electrode and a second capacitive plate or electrode as defined in the second aspect.

Preferably, the device further comprises a first connector configured to be electrically connected to a third connector of a readout device and a second connector configured to be electrically connected to a fourth connector of the readout device, wherein the first connector is electrically connected to a first end of the sensor and the second connector is electrically connected to a second end of the sensor.

For example, the first and second connectors may be a female or male part of a push button and the third and fourth connectors may be a corresponding male or female part of a push button, such that the third connector can be snapped onto the first connector and the fourth connector can be snapped onto the second connector. Also, other forms of connectors are conceivable. For example, the first/third and second/fourth connectors may be structured to provide a plug and socket configuration.

The readout device may be configured to determine a property, preferably an electric property, of the sensor that allows deriving information on the movement of the chest and/or abdomen. For example, the readout device may be configured to determine an inductance, resistance and/or capacity of the sensor. For example, the readout device may be configured to determine a resonance frequency of a resonance circuit, wherein the sensor forms a part of the resonance circuit. Based on the resonance frequency, the readout device and/or a computing device receiving information from the readout device may derive information about the movement of the chest and abdominal and/or information about the user's breathing.

The readout device may be configured to transmit information to a computing device, preferably a portable computing device, e.g., via a wireless connection such as Bluetooth®.

For example, the computing device may be a smartphone, smart watch, smart googles, a smart headset (such as VR, AR, MR googles or headsets), a tablet, notebook or personal computer. Thus, the readout device may comprise a transmitter to transmit the information to a computing device.

Preferably, the first connector is configured to be releasably electrically connected to the third connector and/or the second connector is configured to be releasably electrically connected to the fourth connector.

In this way, the readout device may be removed from the garment, for example, for washing.

Preferably, the first end and the second end of the sensor are both located in one of the first loop and the second loop, i.e., either in the first loop or in the second loop.

That is, the sensor may comprise a longitudinal structure (e.g., a longitudinal conductor), wherein the first end and the second end form opposing ends of the longitudinal structure. These two ends of the sensor may be located in the first or second loop. For example, the sensor may start at the first end, which may be located in a ventral position (i.e. a front position) on the garment, form a first portion (e.g., a first half) of the first loop first loop and extend to a dorsal region (i.e. a back region) of the garment. There, the second loop of the sensor may begin. The sensor may then extend to the ventral region of the garment below the first loop and then extend back to the dorsal region where it crosses and/or overlaps a portion of the sensor at a crossing point. Subsequently, the sensor may form a second portion (e.g., a second half) of the first loop, and the second end of the sensor may then be near the first end of the sensor.

Preferably, the device comprises the readout device.

Preferably, the readout device comprises the third connector configured to be electrically connected to the first connector and the fourth connector configured to be electrically connected to the second connector. Preferably, when the third connector is electrically connected to the first connector and the fourth connector is electrically connected to the second connector, the readout device and the sensor form a resonance circuit.

That is, the sensor may form an inductance of the resonance circuit. Furthermore, the readout device may comprise circuitry that completes the resonance circuit. For example, the circuitry of the readout device may comprise at least one capacitor, which together with the inductance formed by the sensor forms the resonance circuit. Furthermore, the circuitry of the readout device may comprise at least one inductance that also forms part of the resonance circuit.

Preferably, the readout device further comprises readout circuitry for measuring a current and/or a voltage of the resonance circuit.

The readout device may be configured to measure the current and/or voltage over time. By measuring the current and/or voltage, the readout device or a computing device receiving information from the readout device may derive information on the movement of the chest and/or abdomen.

Preferably, the readout circuitry is configured for measuring a resonance frequency of the resonance circuit.

Based on the resonance frequency, the readout circuitry and/or computing device may derive information about the movement of the chest and/or abdomen and/or about chest and/or abdominal breathing. For example, the readout circuitry and/or computing device may be configured to determine an inductance of the sensor, which allows deriving information about the movement of the chest and/or abdomen and/or about the patient's breathing.

A second aspect of the invention relates to a device for sensing a user's respiratory motion of the user's chest and abdomen, comprising a garment configured to be worn on the upper body by the user, a sensor configured to sense the expansion and/or contraction of the user's chest, the sensor being attached to the garment comprising a loop configured to at least partly extend over one of the user's chest and abdomen, and a first capacitive plate or electrode and a second capacitive plate or electrode configured to contact and/or be positioned at least partly on or near the other one of the user's chest and abdomen.

As regards the garment and the sensor, the explanations and additional disclosure provided with regard to the first aspect apply mutatis mutandis.

However, according to the second aspect, the sensor does not need to have a figure eight configuration. Thus, the sensor may comprise only a single loop that is configured to at least partially extend over, preferably to at least partially surround, the user's chest and/or abdomen.

The device according to the second aspect may also further comprise a sensor having a figure eight configuration as defined in the first aspect.

The first capacitive plate or electrode and the second capacitive plate or electrode are configured to be positioned at least partly on the other one of the chest and abdomen. Thus, if the loop is configured to at least partly extend over and/or surround the chest, the first capacitive plate or electrode and the second capacitive plate or electrode are configured to be positioned at least partly on the abdomen. If the loop is configured to at least partly extend over and/or surround the abdomen, the first capacitive plate or electrode and the second capacitive plate or electrode are configured to be positioned at least partly on the chest. In this way, the loop, the first capacitive plate or electrode and the second capacitive plate or electrode may provide a signal or signals that are representative of the movement of the chest and the abdomen. In this way, the device may be configured to sense chest breathing as well as abdominal breathing. Preferably, the first capacitive plate and the second capacitive plate are both configured to be positioned at least partly on a ventral or front region of the chest or abdomen. However, the first capacitive plate may also be configured to be positioned at least partly on a ventral or front region of the chest or abdomen and the second capacitive plate may be configured to be positioned at least partly on a dorsal or back region of the chest or abdomen or vice versa.

Together with the sensor, the first capacitive plate or electrode and the second capacitive plate or electrode may be configured to generate a signal indicating the motion of the user's chest and abdomen. For example, if the device comprises first and second capacitive plates, the capacity of the capacitive plates may vary with the movement of the user's chest and/or abdomen. If the device comprises first and second electrodes, the electric potential of the electrodes may vary with the movement of the user's chest and/or abdomen. Furthermore, a resistance, capacity and/or inductance of the sensor may vary with the movement of the user's chest and/or abdomen. The combination of these varying properties of the sensor and the first and second capacitive plates or electrodes may be exploited to sense the movement of the chest and abdomen, which, in turn allows to sense the patient's breathing.

Such a device can enable measuring the breathing rate, breathing patterns, breathing interruptions, tidal volume, inhale time, exhale time, and/or further breathing-related parameters of a user. Furthermore, the device is able to sense chest breathing and abdominal breathing. Moreover, the sensitivity of the device may be improved and/or motion artifacts in the signal generated by the device may be reduced. Furthermore, the working voltage may be improved and the overall respiration signal amplitude may be increased, which may make the device more robust.

Moreover, the device may allow adapting the contribution of the sensor and the first and second capacitive plates or electrodes, which may enable tuning the device to be more sensitive to chest or abdominal breathing. In this way, the sensing of the user's breathing may be adapted with respect to the user's body position and/or activity level. Furthermore, the device may allow for a calibration of the device, e.g., to improve tidal volume estimation.

Preferably, the first capacitive plate or electrode and the second capacitive plate or electrode are configured to be positioned in the user's abdominal region, preferably a front or ventral abdominal region.

Preferably, the first capacitive plate or electrode is configured to contact the patient's skin, and/or the second capacitive plate or electrode is configured to contact the patient's skin.

Preferably, the first capacitive plate or electrode and/or the second capacitive plate or electrode comprise each has an area between 5cm2 and 100cm2, preferably between 10cm2 and 60cm2, more preferably between 25cm2 and 50 cm2, more preferably around 40cm2.

For example, the first capacitive plate or electrode and/or the second capacitive plate or electrode each has a size of about 5 cm to 8 cm.

It has been found that such a size of the capacitive plates or electrodes is optimal to provide a signal indicative of the movement of the chest and/or abdomen.

Preferably, a distance between the first capacitive plate or electrode and the second capacitive plate or electrode is between 5 cm and 30 cm, preferably between 10 cm and 20 cm.

It has been found that such a spacing between the capacitive plates or electrodes is optimal to provide a signal indicative of the movement of the chest and/or abdomen.

Preferably, at least a portion of a skin-contacting surface of the first capacitive plate or electrode is tacky, and/or at least a portion of a skin-contacting surface of the second capacitive plate or electrode is tacky.

In this way, the first capacitive plate or electrode and/or the second capacitive plate or electrode may have a fixed position with regard to the chest and/or abdomen. In this way, the signal quality of the first and second capacitive plate or electrode may be improved, in particular under movement of the user's body.

Preferably, the device comprises a first tacky skin-contacting surface portion at least partly surrounding the first capacitive plate or electrode, and/or the device comprises a second tacky skin-contacting surface portion at least partly surrounding the second capacitive plate or electrode. More preferably, the first tacky skin-contacting surface is ring-shaped and completely surrounding the first capacitive plate and/or the second tacky skin-contacting surface is ring-shaped and completely surrounding the second capacitive plate.

In this way, the first capacitive plate or electrode and/or the second capacitive plate or electrode may have a fixed position with regard to the chest and/or abdomen. In this way, the signal quality of the first and second capacitive plate or electrode may be improved, in particular under movement of the user's body.

Preferably, the first capacitive plate or electrode and the second capacitive plate or electrode are electrically connected to the sensor.

Preferably, the first capacitive plate or electrode, the second capacitive plate or electrode and the sensor may be read out via a single channel. In other words, it is not necessary to read out and evaluate the signal of the sensor and the signal of the first capacitive plate or electrode and the second capacitive plate or electrode separately. In this way, the readout and analysis of the signal generated by the device may be simplified.

Preferably, the first capacitive plate or electrode, the second capacitive plate or electrode, and the sensor are electrically connected in series.

Preferably, in the electrical connection of the first capacitive plate or electrode, the second capacitive plate or electrode and the sensor, the sensor is arranged between the first capacitive plate or electrode and the second capacitive plate or electrode.

Preferably, the first capacitive plate or electrode, the second capacitive plate or electrode, and the sensor are electrically connected in parallel.

Preferably, the device further comprises a first connector configured to be electrically connected to a third connector of a readout device and a second connector configured to be electrically connected to a fourth connector of the readout device.

Regarding the first connector, the second connector, the third connector and the fourth connector, the explanations and additional disclosure given with respect to the first aspect apply mutatis mutandis.

Preferably, the first connector is configured to be releasably electrically connected to the third connector and/or the second connector is configured to be releasably electrically connected to the fourth connector.

Preferably, both the first connector and the second connector are electrically connected to the first capacitive plate or electrode, the second capacitive plate or electrode and the sensor.

Preferably, the readout device comprises the third connector configured to be electrically connected to the first connector and the fourth connector configured to be electrically connected to the second connector, wherein, when the third connector is electrically connected to the first connector and the fourth connector is electrically connected to the second connector, the readout device, the first capacitive plate or electrode, the second capacitive plate or electrode and the sensor form a resonant circuit.

The readout device may be configured to send information to a computing device, preferably a portable computing device, e.g., via a wireless connection such as Bluetooth®. For example, the computing device may be a smartphone, smart watch, smart googles, smart headset (such as VR, AR, MR googles or headsets), a tablet, notebook or personal computer.

Preferably, the first connector is configured to be releasably electrically connected to the third connector and/or the second connector is configured to be releasably electrically connected to the fourth connector.

In this regard, the explanations and additional disclosure provided with respect to the first aspect apply mutatis mutandis.

Preferably, the readout device comprises at least one inductor and/or at least one capacitor, which are arranged such that the resonant circuit comprises the first capacitive plate or electrode, the second capacitive plate or electrode, the sensor, the at least one inductor and/or the at least one capacitor, when the third connector is electrically connected to the first connector and the fourth connector is electrically connected to the second connector.

Preferably, the readout device comprises a plurality of inductors and a first multiplexer configured to select one or more of the plurality of inductors that is/are arranged in the resonance circuit. The plurality of inductors may be part of the resonance circuit. For example, the plurality of inductors may be arranged in series with the sensor. One of the plurality of inductors may be a short circuit.

Preferably, the readout device comprises a plurality of capacitors and a second multiplexer configured to select one or more of the plurality of capacitors that is/are arranged in the resonance circuit. The plurality of capacitors may be part of the resonance circuit. For example, the plurality of capacitors may be arranged in series or in parallel to the first and second capacitors or electrodes. For example, if the first and second capacitor plates or electrodes and the sensor are arranged in series, the plurality of capacitors may be arranged in series with the first and second capacitor plates or electrodes and the sensor. In turn, if the first and second capacitor plates or electrodes and the sensor are arranged in parallel, the plurality of capacitors may be arranged parallel to the first and second capacitor plates or electrodes and the sensor. One of the plurality of capacitors may be an electrically open loop.

Preferably, the readout device further comprises a control circuitry for controlling the first multiplexer and/or the second multiplexer. The control circuitry may be part of the readout circuitry.

By controlling the first multiplexer and/or the second multiplexer to select one or more of the plurality of inductors and/or one or more of the plurality of capacitors, respectively, the relative contribution of the sensor and the first and second capacitive plate or electrode to the signal can be adapted. For example, the first and/or second multiplexer can be controlled such that the sensor has a stronger contribution to the signal than the first and second capacitor plates or electrodes or vice-versa. In this way, the relative sensitivity of each sensing element (sensor, first capacitive plate or electrode and second capacitive plate or electrode) can be adapted to tune the overall breathing signal and with it the relative sensitivity to chest breathing vs abdominal breathing.

Preferably, the control circuitry is configured to control the sensitivity of the sensor, the first capacitive plate or electrode, and the second capacitive plate or electrode. In other words, the relative contribution of the sensor, the first capacitive plate or electrode and the second capacitive plate or electrode to the signal generated by the device can be controlled.

Preferably, the control circuitry is configured to change a ratio between the sensitivity of the sensor and the sensitivity of the first capacitive plate or electrode and the second capacitive plate or electrode. In other words, a ratio of the contribution of the sensor and a contribution of the first capacitive plate or electrode and the second capacitive plate or electrode to the signal generated by the device can be controlled.

In this way, for example, the control circuitry device can concentrate either on chest or on abdominal breathing. For example, when selecting a capacitor with a high capacitance by the second multiplexer, the contribution of the first and second capacitive plates or electrodes will be smaller and the main signal strength comes from the sensor. Vice-versa, when a capacitor with a smaller capacitance or no capacitor is selected by the second multiplexer, the main signal strength comes from the first and second capacitive plates or electrodes. Likewise, the influence of the sensor on the signal can be adjusted by selecting an inductor with a higher or lower inductance by the first multiplexer. Furthermore, the contribution of the sensor and the first and second capacitive plates or electrodes to the signal may be adjusted to be substantially equal. In this way, abdominal breathing has the same signal impact as chest breathing.

Preferably, the control circuitry is configured to acquire information on a body position of the user and to control the first multiplexer and/or the second multiplexer based on the body position.

Preferably, the information on a body position may be acquired via an accelerometer of the readout device.

In this way, the one or more inductors and/or the one or more capacitors may be selected based on body position of the user. The body position may, e.g., comprise standing, sitting and lying as well as specific lying positions such as belly lying position, back lying position, right side lying position or left side lying position. In this way, the contribution of the sensor and the first and second capacitive plates or electrodes can be adjusted based on the body position so that the amplitude of the signal is adjusted according to the body position. For example, in this way, it may be taken account of the fact that the breathing movement may depend on the body position.

Preferably, the control circuitry is configured to acquire information on an activity level of the user and to control the first multiplexer and/or the second multiplexer based on the activity level and/or on the body position.

Preferably, the information on an activity level may be acquired via an accelerometer of the readout device.

For example, if the control circuitry registers a lower activity level, the first and/or second multiplexer may be controlled so that the main contribution to the signal comes from the first and second capacitive plate or electrode, e.g., by selecting no capacitor or a capacitor with a low capacitance. In turn, when the control circuitry registers a higher activity level, the first and/or second multiplexer may be controlled so that the main contribution to the signal comes from the sensor, since the sensor may yield a more stable signal during movements.

Preferably, the readout device further comprises readout circuitry for measuring a current and/or a voltage of the resonance circuit.

Preferably, the readout circuitry is configured for measuring a resonance frequency of the resonance circuit.

Preferably, the device comprises a first capacitive plate and a second capacitive plate.

Preferably, the first capacitive plate and the second capacitive plate are configured to be electrically isolated from the user's skin in use.

The features described in the following may apply to both the first aspect and the second aspect.

Preferably, the sensor comprises an extensible conductor. Preferably, the conductor wire is elastic.

Preferably, the sensor comprises a resistive strain sensor.

A resistive strain sensor may measure changes in the resistance of the sensor. These changes in the resistance may be measured by measuring changes in the resonance circuit described above. Alternatively, the changes in the resistance of the sensor may be measured via a resistive readout.

The resistive strain sensor may be based on standard strain gauges such as piezoresistive elements or yarns or elastomers with conductive fillers or conductive coatings. They may be in the form of wires, fibers, fabrics, films, foams, usually in elongated form factors.

Preferably, the sensor comprises a capacitive wire sensor.

A capacitive wire sensor may partially or completely surround the body. The capacitance can be measured by measuring changes in the resonance circuit described above. Alternatively, the capacitance may be measured by standard ways to read out a capacitance.

The capacitive sensor changes its capacitance upon elongation and/or pressure. The sensor element may be based on two conductive areas that are electrically insulated from each other. The conductive areas may be based on conductive elastomers, elastomers filled with conductive fillers or conductive coatings any form factors. For example, the sensing element may be based on elastic conductive thin films or conductive mesh structures or stretchable wire or yarns that form a capacitor (similar to a coax cable). The sensor element may be in the form of wires, fibers, fabrics, films, foams, usually in an elongated form factors.

Preferably, the sensor comprises a respiratory plethysmography inductance band. The respiratory plethysmography inductance band may form a coil when arranged in one or more loops. When looped around the upper body of a user, the cross-section of the coil may vary with the movement (in particular expansion and contraction) of the upper body of the user, which varies the inductance of the coil. The inductance of the coil may, e.g., be determined by placing the coil in a resonance circuit and by measuring a property, e.g., a resonance frequency, of the resonance circuit.

Preferably, the extensible conductor comprises an extensible core, preferably a rubber core, and an electrically conductive wire, preferably a Litz wire, wrapped around the extensible core.

Preferably, the extensible conductor comprises an electrically conducive wire arranged in a sinusoid, wave and/or serpentine pattern. Preferably the electrically conductive wire is provided on an extensible piece of material. Said piece of material may, e.g., be part of the garment.

Preferably, the extensible conductor comprises an extensible body, preferably an elastomeric body filled with conductive elements.

Preferably, the garment is a shirt, a bra, in particular a sports bra, or an upper body band or belt.

Preferably, the device further comprises a first electrode and a second electrode attached to the garment, both the first electrode and the second electrode being configured to be in contact with the user's skin at the user's chest and/or abdomen.

The first and second electrode may be configured to provide an ECG signal. Thus, the device may be configured to generate a first signal relating to breathing movement and a second signal relating to the heart's electrical activity, in particular pulse.

Preferably, the device comprises a fifth connector electrically connected to the first electrode and a sixth connector electrically connected to the second electrode, wherein the fifth connector is configured to be electrically connected to a seventh connector of a readout device and the sixth connector configured to be electrically connected to an eighth connector of the readout device.

In this regard, the explanations and disclosure provided with regard to the first to fourth connectors applies to the fifth to eight connectors mutatis mutandis.

Preferably, the fifth connector is configured to be releasably electrically connected to the seventh connector and/or the sixth connector is configured to be releasably electrically connected to the eighth connector.

Preferably, the device comprises the readout device, wherein the readout device is configured to measure the electrical activity of the heart, when the fifth connector is electrically connected to the seventh connector of the readout device and the sixth connector is electrically connected to the eighth connector of the readout device.

Preferably, the resonance frequency of the resonance circuit is between 1 MHz and 10 MHz.

Preferred embodiments of the present invention are further elucidated below with reference to the following figures.

Fig. 1 shows a device for sensing a user's motion of the user's chest and abdomen with a sensor having a figure eight configuration.

Fig. 2 shows a sensor having a figure eight configuration.

Fig. 3 shows a readout device.

Fig. 4 shows a device for sensing a user's motion of the user's chest and abdomen with a sensor, a first capacitive plate or electrode and a second capacitive plate or electrode.

Figs. 5a to 5d show different resonance circuits formed by the device and the readout device.

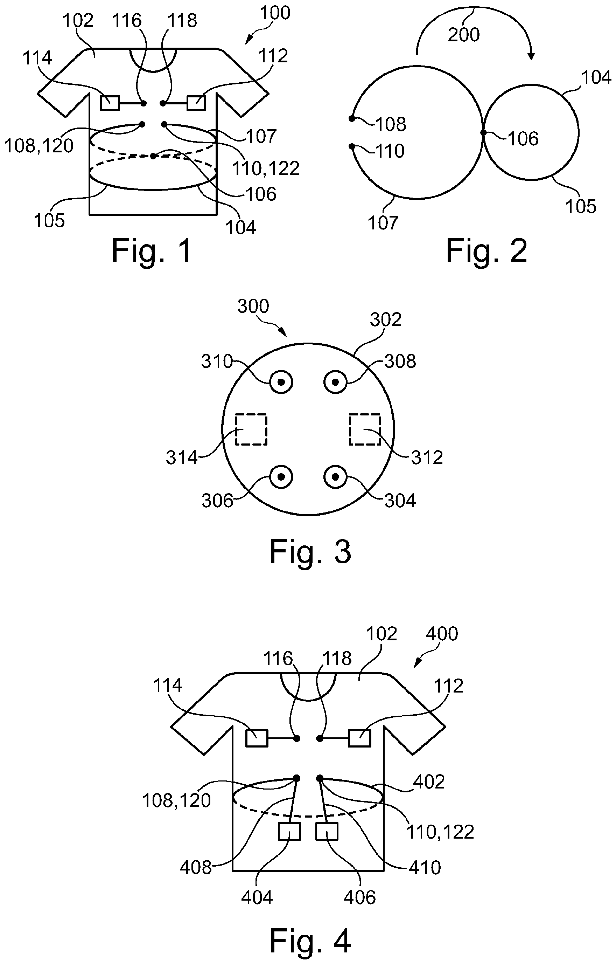

Fig. 1 shows an exemplary embodiment of the device 100 for sensing a user's motion of the user's chest and abdomen according to the first aspect.

The device 100 may comprise a garment 102, e.g., a shirt as shown in Fig. 1. The garment, however, may be another piece of clothing that can be worn at least partially on the upper body. For example, the garment may be a top, a bra, a vest, or an upper body belt.

The device 100 comprises a sensor 104 attached to the garment 102, the sensor 104 having a figure eight configuration. The sensor 100 comprises a first loop 107 configured to at least partially extend along and/or surround the chest and a second loop 105 configured to at last partially extend along and/or surround the abdomen. The first loop 107 and the second loop 105 meet at the crossing point 106, which, preferably, is located at the back.

The first end 108 and the second end 110 of the sensor 104 may be located in the first loop 107, preferably in a ventral region of the garment 102. Furthermore, the device may comprise a first connector 120 and a second connector 122 electrically connected to the first end 108 and the second end 110, respectively. The first connector 120 and the second connector 122 are configured to be connected to respective third connector 304 and fourth connector 306 of a readout device 300 (see Fig. 3).

Furthermore, the device may optionally comprise a first electrode 114 and a second electrode 112 electrically connected to the fifth connector 116 and a sixth connector 118. The fifth connector 116 and the sixth connector 188 are configured to be connected to respective seventh connector 308 and eight connector 310 of a readout device 300 (see Fig. 3).

Fig. 2 shows a sensor 104 in a figure eight configuration, when laid out flat. As can be seen, the sensor 104 forms a first loop 107 and a second loop 105, wherein the first loop 107 adjoins the second loop 105 at the crossing point 106. The crossing point 106 refers to the location where a first portion of the sensor 104 crosses a second portion of the sensor 104, i.e. a first portion of the sensor 104 overlaps a second portion of the sensor 104. When attached to the garment 102 as shown in Fig. 1, the first loop 107 is folded over the second loop 105 as indicated by the arrow 200, such that the first loop 107 and the second loop 105 both encircle a cylinder that fits over an upper body of a user.

Fig. 3 shows a readout device 300, which may be connected to the device 100 shown in Fig. 1 or the device 400 shown in Fig. 4.

The readout device 300 comprises a third connector 304 configured to be releasably connected to the first connector 120 of the device 100, 400 and a fourth connector 306 configured to be releasably connected to the second connector 110 of the device 100, 400.

Furthermore, the readout device 300 may comprise a seventh connector 308 configured to be connected to the fifth connector 116 of the device 100, 400 and an eight connector 310 configured to be connected to the sixth connector 118 of the device 100, 400.

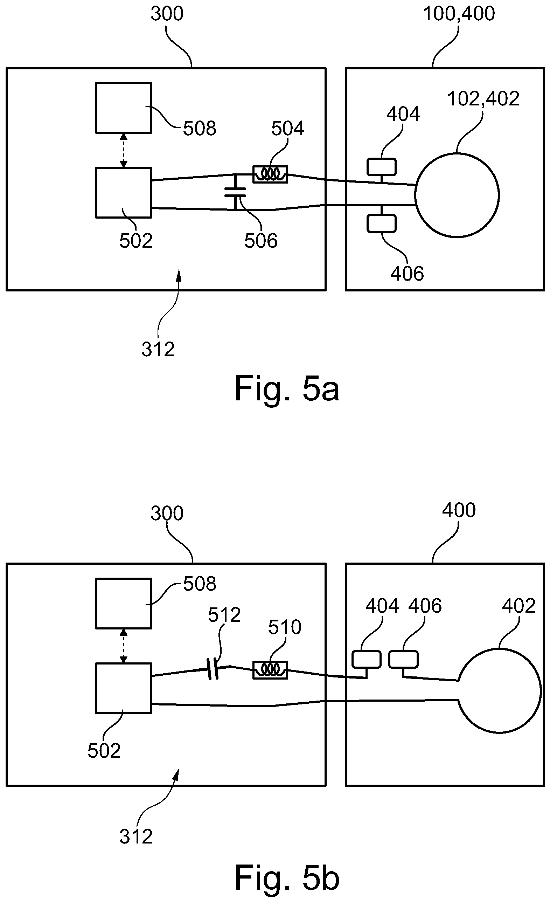

The readout device 300 further comprises readout circuitry 312. The readout circuitry 312 may comprise circuitry that forms a resonance circuit with the sensor 104 of the device 100 or with the sensor 402 and the capacitive plates or electrodes 404, 406 of the device 400. Such resonance circuits are, e.g., shown in Figs. 5a to 5d. Furthermore, the readout circuitry 312 may be configured to readout the signal generated by the first and second electrodes 112, 114 relating to the heart activity.

The readout device 300 may further comprise a transmitter 314 configured to send information to a computing device, e.g., via Bluetooth®.

The readout device 300 may comprise a housing 302 configured to enclose the components of the readout device 300, e.g., the readout circuitry 312 and the transmitter 314, e.g., in a watertight manner.

Fig. 4 shows an exemplary embodiment of the device 400 for sensing a user's motion of the user's chest and abdomen according to the second aspect.

Regarding the components that have the same reference numbers and are equivalent to the components of the device of Fig. 1, reference is made to the description of the embodiment of Fig. 1. In the following, only the components that differ from the embodiment of Fig. 1 are described.

The device 400 comprises a sensor 402 that forms a loop configured to at least partially extend along and/or surround the user's chest. The two ends 108, 110 of the sensor 402 are located at the front of the garment 102. Furthermore, the device 400 comprises a first connector 120 and a second connector 122 electrically connected to a respective one of the first end 108 and the second end 110. Moreover, the device 400 comprises a first capacitive plate or electrode 404 and a second capacitive plate or electrode 406, which are configured to contact the patient's skin at least partially at the patient's abdomen. The first capacitive plate or electrode 404 is electrically connected to the first connector 120 by means of conductor 408 and the second capacitive plate or electrode is electrically connected to the second connector 122 by means of conductor 410.

However, alternatively, the sensor 402 may be configured to at least partially extend along and/or surround the abdomen and the first and second capacitive plates or electrodes 404, 406 may be configured to contact the patient's skin at the patient's chest.

Figs. 5a to 5d show different resonance circuits formed by the device 100, 400 and the readout circuitry 312 of the readout device 300.

The device 100, 400 comprises the sensor 102, 402, the first capacitive plate or electrode 404 and the second capacitive plate or electrode 406. Thus, the device 100, 400 may be the device of the second aspect. However, the device 400 does not necessarily need to have the first and second capacitive plates or electrodes 404, 406, such that the device 100, 400 may be the device of the first aspect.

Fig. 5a shows a first exemplary embodiment of the resonance circuit. According to this exemplary embodiment, the readout circuitry 312 closes the circuit formed by the sensor 102, 402. The resonance circuit may optionally comprise an inductor 504 with an inductance L1 that is arranged in series with the sensor 102, 402. Furthermore, the resonance circuit may optionally comprise a capacitor 506 with a capacity C1 that is arranged in parallel to the sensor 102, 402.

The resonance circuit may comprise an integrated circuit 502 controlled by a microcontroller 508. The microcontroller 508 may read out the measurement results from the integrated circuit 502. It can also change the measurement parameters, like measurement frequency, amplitude, sampling rate, current, voltage used by the IC. The integrated circuit may measure the resonance frequency, the inductance, capacitance, impedance or resistance. When the capacitance is constant in the circuitry, the IC measures inductance change and reciprocally when the inductance is constant, the IC measures capacitance change.

The capacitive plates 404, 406 have a capacity $Cext + \Delta Cext$, wherein $\Delta Cext$ is the change to the capacity $Cext$ of the capacitive plates 404, 406 when the body moves. The sensor has an inductance $Lext + \Delta Lext$, wherein $\Delta Lext$ is the change to the inductance $Lext$ of the sensor when the body moves. The resonance frequency of the resonance circuit is

$$fres = \frac{1}{2\pi\sqrt{(C1+Cext+\Delta Cext)*(L1+Lext+\Delta Lext)}}.$$

Fig. 5b shows a second exemplary embodiment of the resonant circuit. According to this exemplary embodiment, the first and second capacitive plates or electrodes 404, 406 and the sensor 402 are arranged in series. Furthermore, the optional capacitor 512 and the optional inductance 510 of the readout circuitry 312 are arranged in series with the first and second capacitive plates or electrodes 404, 406 and the sensor 402.

In this case, the resonance frequency of the resonant circuit is

$$fres = \frac{1}{2\pi\sqrt{((C1*(Cext+\Delta Cext))/(C1+(Cext+\Delta Cext)))*(L1+Lext+\Delta Lext)}},$$

wherein C1 denotes the capacity of the of the capacitor 512, L1 the inductance of the inductor 510, Cext + ΔCext the capacity of the capacitive plates 404, 406, and Lext + ΔLext the inductance of the sensor 402.

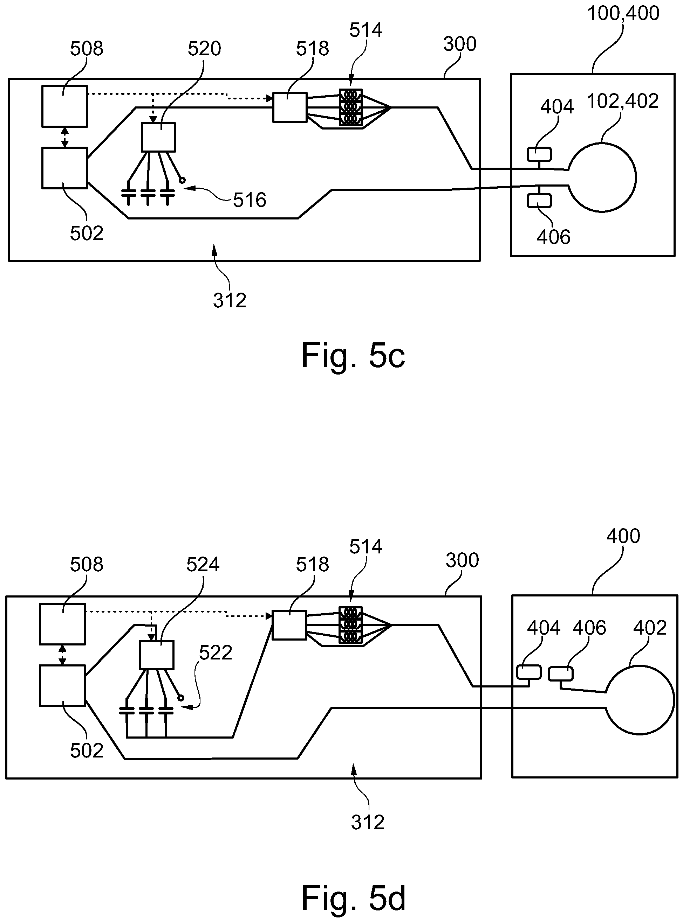

Fig. 5c shows a third exemplary embodiment of the resonant circuit. The third exemplary embodiment differs from the first embodiment in that the readout circuitry 312 comprises a plurality of inductances 514 and a plurality of capacitors 516. The plurality of inductances 514 is arranged in series with the sensor 102, 402 and the plurality of capacitors 516 is arranged in parallel to the sensor 102, 402. The plurality of inductors 514 can have inductances L1 to Ln, wherein one of the inductances Lx is a short-circuit. Furthermore, the plurality of capacitors 516 can have the capacities C1 to Cn, wherein one of the capacities Cx is an electrically open loop.

Furthermore, the readout circuitry 312 comprises a first multiplexer 518 configured to select one or more of the plurality of inductors 514, wherein one of the plurality of inductors 514 can be a short-circuit. The readout circuitry 312 further comprises a second multiplexer 520 configured to select one or more of the plurality of capacitors 516, wherein one of the plurality of capacitors 516 can be an electrically open loop. The inductor selected by the first multiplexer 518 has an inductance Lx and the capacitor selected by the second multiplexer 520 has a capacity Cx.

However, there may also be embodiments with only one multiplexer, e.g., with only the first multiplexer 518 configured to select one or more of the plurality of inductors 514, or with only the second multiplexer 520 configured to select one or more of the plurality of capacitors Cx.

The microcontroller 508 is configured to control the first multiplexer 518 and/or the second multiplexer 520. For example, the microcontroller 508 is configured to receive information regarding a body position and/or activity level, e.g., from an accelerometer. Furthermore, the microcontroller 508 may control the first multiplexer 518 and/or the second multiplexer 520 based on said information.

In this case, the resonance frequency of the resonant circuit is

$$fres = \frac{1}{2\pi\sqrt{(Cx+Cext+\Delta Cext)*(Lx+Lext+\Delta Lext)}},$$

wherein Cext + ΔCext is the capacity of the capacitive plates 404, 406, and Lext + ΔLext is the inductance of the sensor 402.

Fig. 5d shows a fourth exemplary embodiment of the resonant circuit. The fourth exemplary embodiment differs from the second embodiment in that the readout circuitry 312 comprises a plurality of inductances 514 and a plurality of capacitors 522. The plurality of inductances 514 is arranged in series with the sensor 402 and the plurality of capacitors 522 is arranged in parallel to the sensor 102, 402. The plurality of inductors 514 can have inductances L1 to Ln, wherein one of the inductances Lx can be a short circuit. Furthermore, the plurality of capacitors 522 can have the capacities C1 to Cn, wherein one of the capacities Cx can be an electrically open loop.

Furthermore, the readout circuitry 312 comprises a first multiplexer 518 configured to select one or more of the plurality of inductors 514, wherein one of the plurality of inductors 514 can have be a short circuit. The readout circuitry 312 further comprises a second multiplexer 524 configured to select one or more of the plurality of capacitors 522, wherein one of the plurality of capacitors 522 can be an electrically open loop. The inductor selected by the first multiplexer 518 has an inductance Lx and the capacitor 522 selected by the second multiplexer 524 has a capacity Cx.

However, there may also be embodiments with only one multiplexer, e.g., with only the first multiplexer 518 configured to select one or more of the plurality of inductors 514, or with only the second multiplexer 524 configured to select one or more of the plurality of capacitors 522. The microcontroller 508 is configured to control the first multiplexer 518 and/or the second multiplexer 524. For example, the microcontroller 508 is configured to receive information regarding a body position and/or activity level, e.g., from an accelerometer. Furthermore, the microcontroller 508 may control the first multiplexer 518 and/or the second multiplexer 524 based on said information.

In this case, the resonance frequency of the resonant circuit is

$$fres = \frac{1}{2\pi\sqrt{((Cx*(Cext+\Delta Cext))/(Cx+(Cext+\Delta Cext)))*(L1+Lext+\Delta Lext)}},$$

wherein Cext + ΔCext the capacity of the capacitive plates 404, 406, and Lext + ΔLext the inductance of the sensor 402.

Further embodiments of the invention are disclosed in the following aspects:

1. A device for sensing a user's motion of the user's chest and abdomen, comprising:

a garment configured to be worn on the upper body by the user; and

a sensor configured to sense the expansion and/or contraction of the user's chest and abdomen, the sensor being attached to the garment,

wherein the sensor has a figure eight configuration with a first loop of the sensor configured to at least partly extend over and/or surround the user's chest, a second loop of the sensor configured to at least partly extend over and/or surround the user's abdomen;

wherein, preferably, device is configured to be worn on the upper body of the user such that a crossing point between the first loop and the second loop is located at the user's back.

2. The device of any one of the preceding aspects, further comprising:

a first connector configured to be electrically connected to a third connector of a readout device and a second connector configured to be electrically connected to a fourth connector of the readout device,

wherein the first connector is electrically connected to a first end of the sensor and the second connector is electrically connected to a second end of the sensor.

3. The device of aspect 2, wherein the first connector is configured to be releasably electrically connected to the third connector and/or the second connector is configured to be releasably electrically connected to the fourth connector.

4. The device of aspect 2 or 3,
wherein the first end and the second end of the sensor are both located in one of the first loop and the second loop.

5. The device of any one of aspects 2 to 4, further comprising:

the readout device comprising the third connector configured to be electrically connected to the first connector and the fourth connector configured to be electrically connected to the second connector,

wherein, when the third connector is electrically connected to the first connector and the fourth connector is electrically connected to the second connector, the readout device and the sensor form a resonance circuit.

6. The device of aspect 5,
wherein the readout device further comprises readout circuitry for measuring a current and/or a voltage of the resonance circuit.

7. The device of aspect 6, wherein the readout circuitry is configured for measuring a resonance frequency of the resonance circuit.

8. A device for sensing a user's respiratory motion of the user's chest and abdomen, comprising:

a garment configured to be worn on the upper body by the user;

a sensor configured to sense the expansion and/or contraction of the user's chest, the sensor being attached to the garment comprising a loop configured to at least partly extend over and/or surround one of the user's chest and abdomen;

a first capacitive plate or electrode and a second capacitive plate or electrode configured to contact and/or be positioned at least partly on or near the other one of the user's chest and abdomen.

9. The device of aspect 8, wherein the first capacitive plate or electrode and the second capacitive plate or electrode are configured to be positioned in the user's abdominal region, preferably a front abdominal region.

10. The device of aspect 8 or 9,

wherein the first capacitive plate or electrode is configured to contact the patient's skin; and/or
wherein the second capacitive plate or electrode is configured to contact the patient's skin.

11. The device of any one of aspects 8 to 10,

wherein the first capacitive plate or electrode and/or the second capacitive plate or electrode comprise each has an area between 5cm$^2$ and 100cm$^2$, preferably between 10cm$^2$ and 60cm$^2$, more preferably between 25cm$^2$ and 50 cm$^2$, more preferably around 40cm$^2$; and/or

wherein a distance between the first capacitive plate or electrode and the second capacitive plate or electrode is between 5cm and 30cm, preferably between 10cm and 20cm.

12. The device of any one of aspects 8 to 11,

wherein at least a portion of a skin-contacting surface of the first capacitive plate or electrode is tacky; and/or
wherein at least a portion of a skin-contacting surface of the second capacitive plate or electrode is tacky.

13. The device of any one of aspects 8 to 12, wherein the device comprises a first tacky skin-contacting surface portion at least partly surrounding the first capacitive plate or electrode; and/or
wherein the device comprises a second tacky skin-contacting surface portion at least partly surrounding the second capacitive plate or electrode.

14. The device of any one of aspects 7 to 13,
wherein the first capacitive plate or electrode and the second capacitive plate or electrode are electrically connected to the sensor.

15. The device of aspect 14,
wherein the first capacitive plate or electrode, the second capacitive plate or electrode, and the sensor are electrically connected in series.

16. The device of aspect 14,
wherein the first capacitive plate or electrode, the second capacitive plate or electrode, and the sensor are electrically connected in parallel.

17. The device of aspect 15,
wherein, in the electrical connection of the first capacitive plate or electrode, the second capacitive plate or electrode and the sensor, the first capacitive plate or electrode and the second capacitive plate or electrode are arranged between the first end and the second end of the sensor.

18. The device of any one of aspects 8 to 17, further comprising

a first connector configured to be electrically connected to a third connector of a readout device and a second connector configured to be electrically connected to a fourth connector of the readout device;
wherein, preferably, the first connector is configured to be releasably electrically connected to the third connector and/or the second connector is configured to be releasably electrically connected to the fourth connector.

19. The device of aspect 18,
wherein both the first connector and the second connector are electrically connected to the first capacitive plate or electrode, the second capacitive plate or electrode and the sensor.

20. The device of aspect 18 or 19, further comprising

the readout device comprising the third connector configured to be electrically connected to the first connector and the fourth connector configured to be electrically connected to the second connector,
wherein, when the third connector is electrically connected to the first connector and the fourth connector is electrically connected to the second connector, the readout device, the first capacitive plate or electrode, the second capacitive plate or electrode and the sensor form a resonant circuit.

21. The device of aspect 20, wherein the first connector is configured to be releasably electrically connected to the third connector and/or the second connector is configured to be releasably electrically connected to the fourth connector.

22. The device of aspect 20 or 21,
wherein the readout device comprises at least one inductor and/or at least one capacitor, which are arranged such that the resonant circuit comprises the first capacitive plate or electrode, the second capacitive plate or electrode, the sensor, the at least one inductor and/or the at least one capacitor, when the third connector is electrically connected to the first connector and the fourth connector is electrically connected to the second connector.

23. The device of aspect 22,

wherein the readout device comprises a plurality of inductors and a first multiplexer configured to select one or more of the plurality of inductors that is/are arranged in the resonant circuit.

24. The device of aspect 22 or 23,
wherein the readout device comprises a plurality of capacitors and a second multiplexer configured to select one or more of the plurality of capacitors that is/are arranged in the resonant circuit.

25. The device of aspect 23 or 24,
wherein the readout device further comprises a control circuitry for controlling the first multiplexer and/or the second multiplexer.

26. The device of aspect 25,
wherein the control circuitry is configured to control the sensitivity of the sensor, the first capacitive plate or electrode, and the second capacitive plate or electrode.

27. The device of aspect 25 or 26,
wherein the control circuitry is configured to change a ratio between the sensitivity of the sensor and the sensitivity of the first capacitive plate or electrode and the second capacitive plate or electrode.

28. The device of any one of aspects 25 to 27, wherein the control circuitry is configured to acquire information on a body position of the user and to control the first multiplexer and/or the second multiplexer based on the body position.

29. The device of any one of aspects 25 or 28, wherein the control circuitry is configured to acquire information on an activity level of the user and to control the first multiplexer and/or the second multiplexer based on the activity level of the user.

30. The device of any one of aspects 20 to 29,
wherein the readout device further comprises readout circuitry for measuring a current and/or a voltage of the resonance circuit.

31. The device of aspect 30,
wherein the readout circuitry is configured for measuring a resonance frequency of the resonance circuit.

32. The device of any one of aspects 8 to 31, comprising a first capacitive plate and a second capacitive plate.

33. The device of aspect 32, wherein the first capacitive plate and the second capacitive plate are configured to be electrically isolated from the user's skin in use.

34. The device of any one of the preceding aspects, wherein the sensor comprises an extensible conductor.

35. The device of any one of the preceding aspects, wherein the sensor comprises a resistive strain sensor.

36. The device of any one of the preceding aspects, wherein the sensor comprises a capacitive wire sensor.

37. The device of any one of the preceding aspects, wherein the sensor comprises a respiratory plethysmography inductance band.

38. The device of any one of the preceding aspects,
wherein the extensible conductor comprises:

- an extensible core, preferably a rubber core, and an electrically conductive wire, preferably a Litz wire, wrapped around the extensible core;
- an electrically conductive wire arranged in a sinusoid, wave and/or serpentine pattern; or
- an extensible body, preferably an elastomeric body filled with conductive elements.

39. The device of any one of the preceding aspects,
wherein the garment is a shirt, a bra, in particular a sports bra, or an upper body band or belt.

40. The device of any one of the preceding aspects, further comprising:
a first electrode and a second electrode attached to the garment, both the first electrode and the second electrode being configured to be in contact with the user's skin at the user's chest and/or abdomen.

41. The device of aspect 40, further comprising:

a fifth connector electrically connected to the first electrode and a sixth connector electrically connected to the second electrode;
wherein the fifth connector is configured to be electrically connected to a seventh connector of a readout device and the sixth connector configured to be electrically connected to an eighth connector of the readout device; and wherein, preferably, the fifth connector is configured to be releasably electrically connected to the seventh connector and/or the sixth connector is configured to be releasably electrically connected to the eighth connector.

42. The device of aspect 41, further comprising:

the readout device,
wherein the readout device is configured to measure the electrical activity of the heart, when the fifth connector is electrically connected to the seventh connector of the readout device and the sixth connector is electrically connected to the eighth connector of the readout device.

43. The device of any one of aspects 7 and 31, wherein the resonance frequency of the resonance circuit is between 1MHz and 10MHz.

**Claims**

1. A device for sensing a user's motion of the user's chest and abdomen, comprising:

   a garment configured to be worn on the upper body by the user; and
   a sensor configured to sense the expansion and/or contraction of the user's chest and abdomen, the sensor being attached to the garment,
   wherein the sensor has a figure eight configuration with a first loop of the sensor configured to at least partly extend over and/or surround the user's chest, a second loop of the sensor configured to at least partly extend over and/or surround the user's abdomen;
   wherein, preferably, the device is configured to be worn on the upper body of the user such that a crossing point between the first loop and the second loop is located at the user's back.

2. The device of claim 1, further comprising:

   a first connector configured to be electrically connected to a third connector of a readout device and a second connector configured to be electrically connected to a fourth connector of the readout device,
   wherein the first connector is electrically connected to a first end of the sensor and the second connector is electrically connected to a second end of the sensor,
   wherein the first connector is preferably configured to be releasably electrically connected to the third connector and/or the second connector is configured to be releasably electrically connected to the fourth connector,
   wherein the readout device preferably comprises the third connector configured to be electrically connected to the first connector and the fourth connector configured to be electrically connected to the second connector,
   wherein, when the third connector is electrically connected to the first connector and the fourth connector is electrically connected to the second connector, the readout device and the sensor preferably form a resonance circuit.

3. The device of claim 2,
   wherein the readout device further comprises readout circuitry for measuring a current and/or a voltage of the resonance circuit, wherein the readout circuitry is preferably configured for measuring a resonance frequency of the resonance circuit.

4. A device for sensing a user's respiratory motion of the user's chest and abdomen, comprising:

a garment configured to be worn on the upper body by the user;

a sensor configured to sense the expansion and/or contraction of the user's chest, the sensor being attached to the garment comprising a loop configured to at least partly extend over and/or surround one of the user's chest and abdomen;

a first capacitive plate or electrode and a second capacitive plate or electrode configured to contact and/or be positioned at least partly on or near the other one of the user's chest and abdomen.

5. The device of claim 4, wherein the first capacitive plate or electrode and the second capacitive plate or electrode are configured to be positioned in the user's abdominal region, preferably a front abdominal region.

6. The device of any one of claims 4 to 5,

wherein at least a portion of a skin-contacting surface of the first capacitive plate or electrode is tacky; and/or

wherein at least a portion of a skin-contacting surface of the second capacitive plate or electrode is tacky.

7. The device of any one of claims 4 to 6, wherein the device comprises a first tacky skin-contacting surface portion at least partly surrounding the first capacitive plate or electrode; and/or

wherein the device comprises a second tacky skin-contacting surface portion at least partly surrounding the second capacitive plate or electrode.

8. The device of any one of claims 4 to 7,

wherein the first capacitive plate or electrode and the second capacitive plate or electrode are electrically connected to the sensor, wherein the first capacitive plate or electrode, the second capacitive plate or electrode, and the sensor are electrically connected in series, wherein, in the electrical connection of the first capacitive plate or electrode, the second capacitive plate or electrode and the sensor, the first capacitive plate or electrode and the second capacitive plate or electrode are arranged between the first end and the second end of the sensor.

9. The device of any one of claims 4 to 8, further comprising

a first connector configured to be electrically connected to a third connector of a readout device and a second connector configured to be electrically connected to a fourth connector of the readout device;

wherein, preferably, the first connector is configured to be releasably electrically connected to the third connector and/or the second connector is configured to be releasably electrically connected to the fourth connector,

wherein the readout device preferably comprises the third connector configured to be electrically connected to the first connector and the fourth connector configured to be electrically connected to the second connector,

wherein, when the third connector is electrically connected to the first connector and the fourth connector is electrically connected to the second connector, the readout device, the first capacitive plate or electrode, the second capacitive plate or electrode and the sensor preferably form a resonant circuit.

10. The device of claim 9,

wherein the readout device comprises at least one inductor and/or at least one capacitor, which are arranged such that the resonant circuit comprises the first capacitive plate or electrode, the second capacitive plate or electrode, the sensor, the at least one inductor and/or the at least one capacitor, when the third connector is electrically connected to the first connector and the fourth connector is electrically connected to the second connector.

11. The device of claim 10,

wherein the readout device comprises a plurality of inductors and a first multiplexer configured to select one or more of the plurality of inductors that is/are arranged in the resonant circuit,

wherein the readout device preferably comprises a plurality of capacitors and a second multiplexer configured to select one or more of the plurality of capacitors that is/are arranged in the resonant circuit,

wherein the readout device preferably further comprises a control circuitry for controlling the first multiplexer and/or the second multiplexer.

12. The device of claim 11,

wherein the control circuitry is configured to change a ratio between the sensitivity of the sensor and the sensitivity of the first capacitive plate or electrode and the second capacitive plate or electrode.

13. The device of any one of claims 11 to 12, wherein the control circuitry is configured to acquire information on a body position of the user and to control the first multiplexer and/or the second multiplexer based on the body position, wherein the control circuitry is preferably configured to acquire information on an activity level of the user and to control the first multiplexer and/or the second multiplexer based on the activity level of the user.

14. The device of any one of the preceding claims,
wherein the extensible conductor comprises:

• an extensible core, preferably a rubber core, and an electrically conductive wire, preferably a Litz wire, wrapped around the extensible core;
• an electrically conductive wire arranged in a sinusoid, wave and/or serpentine pattern; or
• an extensible body, preferably an elastomeric body filled with conductive elements.

15. The device of any one of the preceding claims, further comprising:
a first electrode and a second electrode attached to the garment, both the first electrode and the second electrode being configured to be in contact with the user's skin at the user's chest and/or abdomen.

16. The device of claim 15, further comprising:

a fifth connector electrically connected to the first electrode and a sixth connector electrically connected to the second electrode;
wherein the fifth connector is configured to be electrically connected to a seventh connector of a readout device and the sixth connector configured to be electrically connected to an eighth connector of the readout device; and
wherein, preferably, the fifth connector is configured to be releasably electrically connected to the seventh connector and/or the sixth connector is configured to be releasably electrically connected to the eighth connector, wherein the device preferably further comprises the readout device,
wherein the readout device is preferably configured to measure the electrical activity of the heart, when the fifth connector is electrically connected to the seventh connector of the readout device and the sixth connector is electrically connected to the eighth connector of the readout device.

Fig. 1

Fig. 2

Fig. 3

Fig. 4

Fig. 5a

Fig. 5b

Fig. 5c

Fig. 5d

**EP 4 520 262 A1**

Europäisches Patentamt

European Patent Office

Office européen des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2011/087115 A1 (SACKNER MARVIN A [US] ET AL) 14 April 2011 (2011-04-14) * paragraphs [0027], [0080]; figure 1 * ----- | 1-3, 14-16 | INV. A61B5/053 A61B5/113 A61B5/00 |
| X | US 2020/397382 A1 (VOLPE SHANE S [US] ET AL) 24 December 2020 (2020-12-24) | 4,5,8-16 | |
| Y | * paragraphs [0047] – [0053]; figure 2 * ----- | 6,7 | |
| Y | US 2008/064964 A1 (NAGATA SHINYA [JP] ET AL) 13 March 2008 (2008-03-13) * paragraph [0097]; figure 1 * ----- | 6,7 | |
| X | EP 3 736 528 B1 (NOKIA TECHNOLOGIES OY [FI]) 17 May 2023 (2023-05-17) * paragraphs [0117], [0223]; figure 10 * ----- | 4,5,8-16 | |

| | TECHNICAL FIELDS SEARCHED (IPC) |
|---|---|
| | **A61B** |

The present search report has been drawn up for all claims

| Place of search | Date of completion of the search | Examiner |
|---|---|---|
| Berlin | 22 February 2024 | Clevorn, Jens |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt

European Patent Office

Office européen des brevets

**Application Number**

EP 23 19 6254

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

**see sheet B**

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION**
**SHEET B**

The Search Division considers that the present European patent application does not comply with the
requirements of unity of invention and relates to several inventions or groups of inventions, namely:

```
1. claims: 1-3(completely); 14-16(partially)

   The sensor has a figure eight configuration with a first
   loop of the sensor configured to at least partly extend over
   and/or surround the user's chest, a second loop of the
   sensor configured to at least partly extend over and/or
   surround the user's abdomen.
                        ---


2. claims: 4-13(completely); 14-16(partially)

   A first capacitive plate or electrode and a second
   capacitive plate or electrode configured to contact and/or
   be positioned at least partly on or near the other one of
   the user's chest and abdomen.
                        ---
```

## ANNEX TO THE EUROPEAN SEARCH REPORT
## ON EUROPEAN PATENT APPLICATION NO.

EP 23 19 6254

This annex lists the patent family members relating to the patent documents cited in the above-mentioned European search report.
The members are as contained in the European Patent Office EDP file on
The European Patent Office is in no way liable for these particulars which are merely given for the purpose of information.

22-02-2024

| Patent document cited in search report | | Publication date | Patent family member(s) | | Publication date |
|---|---|---|---|---|---|
| US 2011087115 | A1 | 14-04-2011 | AU | 5359901 A | 30-10-2001 |
| | | | CA | 2405848 A1 | 25-10-2001 |
| | | | EP | 1296591 A2 | 02-04-2003 |
| | | | EP | 2324760 A2 | 25-05-2011 |
| | | | EP | 2324761 A2 | 25-05-2011 |
| | | | IL | 152300 A | 19-08-2007 |
| | | | JP | 5084923 B2 | 28-11-2012 |
| | | | JP | 2003530184 A | 14-10-2003 |
| | | | JP | 2010279715 A | 16-12-2010 |
| | | | JP | 2011136182 A | 14-07-2011 |
| | | | KR | 20030066322 A | 09-08-2003 |
| | | | US | 9750429 B1 | 05-09-2017 |
| | | | US | 2002032386 A1 | 14-03-2002 |
| | | | US | 2003135127 A1 | 17-07-2003 |
| | | | US | 2011087115 A1 | 14-04-2011 |
| | | | WO | 0178577 A2 | 25-10-2001 |
| US 2020397382 | A1 | 24-12-2020 | BR | 112014003953 A2 | 13-06-2017 |
| | | | CN | 103764222 A | 30-04-2014 |
| | | | CN | 105661695 A | 15-06-2016 |
| | | | EP | 2750761 A1 | 09-07-2014 |
| | | | JP | 2014526282 A | 06-10-2014 |
| | | | KR | 20140057627 A | 13-05-2014 |
| | | | US | 2013085538 A1 | 04-04-2013 |
| | | | US | 2014206974 A1 | 24-07-2014 |
| | | | US | 2015164427 A1 | 18-06-2015 |
| | | | US | 2018132792 A1 | 17-05-2018 |
| | | | US | 2020397382 A1 | 24-12-2020 |
| | | | US | 2023355183 A1 | 09-11-2023 |
| | | | WO | 2013033238 A1 | 07-03-2013 |
| US 2008064964 | A1 | 13-03-2008 | CN | 1933777 A | 21-03-2007 |
| | | | EP | 1731094 A1 | 13-12-2006 |
| | | | EP | 2324764 A2 | 25-05-2011 |
| | | | HK | 1094667 A1 | 04-04-2007 |
| | | | JP | 4698582 B2 | 08-06-2011 |
| | | | JP | 5313272 B2 | 09-10-2013 |
| | | | JP | 2011098214 A | 19-05-2011 |
| | | | JP | WO2005089645 A1 | 31-01-2008 |
| | | | US | 2008064964 A1 | 13-03-2008 |
| | | | WO | 2005089645 A1 | 29-09-2005 |
| EP 3736528 | B1 | 17-05-2023 | EP | 3736528 A1 | 11-11-2020 |
| | | | US | 2022183627 A1 | 16-06-2022 |
| | | | WO | 2020225039 A1 | 12-11-2020 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 1596298 A

- WO 202109428 A1